(19) Europäisches Patentamt
European Patent Office
Office européen des brevets

(11) EP 1 424 078 A1

(12) **EUROPEAN PATENT APPLICATION**
published in accordance with Art. 158(3) EPC

(43) Date of publication:
**02.06.2004 Bulletin 2004/23**

(21) Application number: **02772831.0**

(22) Date of filing: **03.09.2002**

(51) Int Cl.[7]: **A61K 45/00**, A61K 31/437, A61P 11/00, A61P 11/06

(86) International application number:
**PCT/JP2002/008926**

(87) International publication number:
**WO 2003/020313 (13.03.2003 Gazette 2003/11)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **04.09.2001 JP 2001266595**

(71) Applicant: **ONO PHARMACEUTICAL CO., LTD.**
**Osaka-shi, Osaka 541-8526 (JP)**

(72) Inventors:
- **NAKADE, Shinji, Ono Pharmaceutical Co., Ltd.**
  **Mishima-gun, Osaka 618-8585 (JP)**
- **SUZUKI, Hidehiro,**
  **Ono Pharmaceutical Co., Ltd.**
  **Mishima-gun, Osaka 618-8585 (JP)**

(74) Representative: **Henkel, Feiler & Hänzel**
**Möhlstrasse 37**
**81675 München (DE)**

(54) **REMEDIES FOR RESPIRATORY DISEASES COMPRISING SPHINGOSINE-1-PHOSPHATE RECEPTOR CONTROLLER**

(57) Pharmaceutical composition for reducing airway resistance comprising a sphingosine-1-phosphate receptor modulator. Since airway obstruction is enhanced by inhalation of S1P, a S1P receptor antagonist reduces airway resistance, and is useful in treating or preventing for bronchial asthma and chronic obstructive pulmonary disease. And an experimental procedure using a S1P receptor agonist is useful in screening for S1P receptor antagonist.

Figure. 7

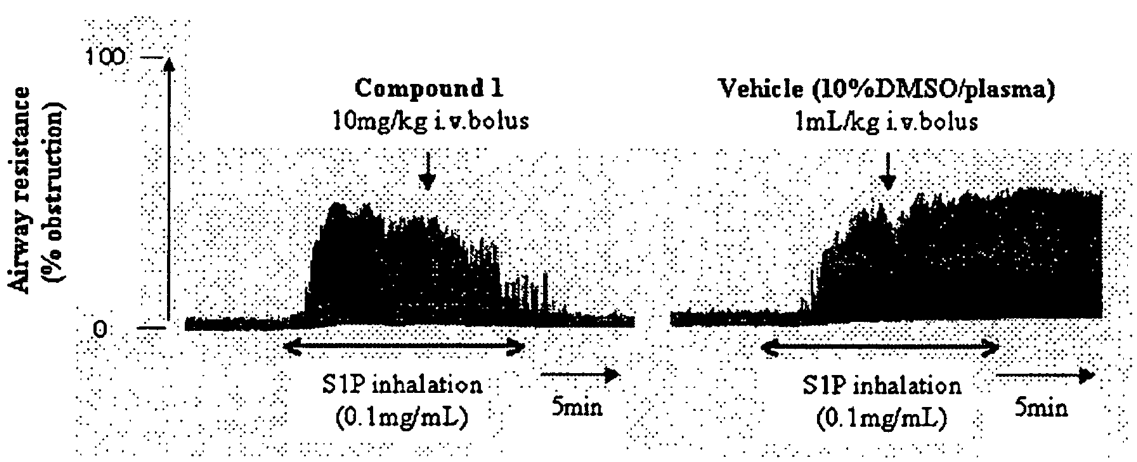


EP 1 424 078 A1

## Description

TECHNICAL FIELD

The present invention relates to a pharmaceutical composition for treatment for respiratory disease comprising a sphingosine-1-phosphate (called S1P or SPP, hereinafter, in the present invention, sometimes abbreviated to as S1P) receptor modulator.

Particularly, the present invention relates to

(1) a pharmaceutical composition for reducing airway resistance or a pharmaceutical composition for growth-inhibition of airway smooth muscle cells comprising a S1P receptor antagonist, and
(2) a method for screening for compounds which have an ability for reducing airway resistance characterized by using S1P receptor agonist.

BACKGROUND ART

Sphingosine-1-phosphate ((2S, 3R, 4E)-2-amino-3-hydroxyoctadeca-4-enyl-1phosphate ; S1P) represented by formula (I)

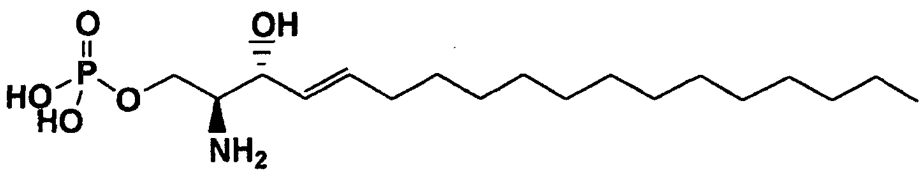

**(I)**

is a lipid which is produced by intracellular sphingolipid turnover or by activities of extracellular secretable sphingosine kinase. It has been supposed that S1P acts as an intercellular or intracellular messenger (Hla et al., Biochem. Pharm. 58, 201 (1999)). At first, an experimental result which suggests S1P acts as an intracellular second messenger was reported (Ghosh et al., Science, 248, 1653 (1990)). But the intracellular molecule of which S1P does direct action has not yet been discovered. On the other hand, it has been reported that EDG-1 (Endothelial Differentiation Gene-1), a G-protein coupled receptor, and it's family molecules EDG-3, EDG-5, EDG-6 and EDG-8 are involved in the extracellular activities of S1P. And it has been known that various pharmacological events occurs via these cell membrane S1P receptors. It has been reported in 1998, that EDG-1 is S1P receptor (Lee et al., Science 279, 1552 (1998)). And then, it has been reported that the other receptors acts as a S1P receptor. It has been disclosed that these receptors have high homology with LPA receptor, EDG-2, 4 and 7. Now, it has been thought that EDG-1-8 form S1P/LPA receptor family.

EDG-1, 3, 5, 6 and 8 are called S1P1, S1P3, S1P2, S1P4 and S1P5, respectively.

As the *in vitro* effects of S1P, cell motility inhibition of smooth muscle cells and cancer cells, platelet aggregation, and the like are known. As the *in vivo* effects of S1P, vascularization, decrease of renal blood flow, inhibition of pulmonary fibrosis, and the like are known. About vascularization effect of S1P, Menq-Jer Lee et al. reported that S1P induces the survival of HUVEC, forming of adhesion plaques and vascularization of small blood vessel via EDG-1 and EDG-3 in human umbilical vein endothelial cells (HUVEC) (Cell 99, 301 (1999)). And also, they reported that S1P has a synergetic effect with FGF (Fibroblast growth factor) or VEGF (Vascular endotherial growth factor) on vascularization *in vivo.* OK-Hee Lee et al. reported that S1P accelerates DNA synthesis and induces cell migration in HUVEC, and S1P induces vascularization by itself *in vivo* (Biochem. Biophys. Res. Commun., 264, 743 (1999)). From these reports, it is thought that vascularization is one of the physiological activities of S1P.

About the effect of S1P on renal blood flow, Angela Bischoff et al. reported recently that S1P induces strong decrease of renal blood flow of rat transiently (Br. J . Pharmacol., 130, 1878 (2000)). It is thought that this action is via S1P receptor bound with Giα, because it is inhibited by pertussis toxin which inhibits a Giα signal.

On the other hand, as effect of S1P on lung, induction effect of cell growth of airway smooth muscle cells (ASM) has been known. Moreover, it has been disclosed that S1P inhibits pulmonary fibrosis in bleomycin-induced pulmonary fibrosis model.

There is no report, however, as to the activity of S1P on an induction of airway resistance. Moreover, there is no report that which receptor is involved in acceleration of cell growth of airway smooth muscle cells by S1P.

As a ligand for S1P receptors, WO01/98301 discloses that pyrazolopyridine compounds which have an antagonistic effect to S1P receptors and those are useful in treatment for hepatic fibrosis, pulmonary fibrosis, renal fibrosis and arterial sclerosis.

DISCLOSURE OF THE INVENTION

The present inventors have studied on physiological activities of S1P in various ways in order to elucidate the role of S1P receptors and found unexpectedly that they are involved in increase of airway resistance. In addition, they have found that EDG-3 and/or EDG-5 among the sub-type of S1P receptors is particularly involved in it. And they have found that EDG-5 antagonist has an inhibitory effect of growth of airway smooth muscle cells, too. These were first ascertained by the present inventors in their experiment, though not expected from the prior art in the least.

Thus, the present invention relates to a pharmaceutical composition for treatment for respiratory disease comprising a S1P receptor modulator.

Particularly, the present invention relates to

1. A pharmaceutical composition for treatment and/or prevention for respiratory diseases comprising a sphingosine-1-phosphate (S1P) receptor modulator.
2. A pharmaceutical composition for treatment and/or prevention for airway constriction, bronchial asthma, chronic obstructive pulmonary disease (COPD), pulmonary emphysema, tracheostenosis, diffused panbronchialitis, or bronchitis with infection, connective-tissue diseases or trasnplantation, lymphangioleiomyomatosis, adult respiratory distress syndrome (ARDS), interstitial pneumonia, lung cancer, hypersensitivity pneumonitis or idiopathic interstitial pneumonia, which comprises the sphingosine-1-phosphate (S1P) receptor modulator according to above-mentioned 1 as an active ingredient.
3. The pharmaceutical composition for treatment and/or prevention for respiratory diseases according to above-mentioned 1, which comprises a S1P receptor modulator having an ability of regulating airway resistance.
4. The pharmaceutical composition for treatment and/or prevention for respiratory diseases according to above-mentioned 1, which comprises a S1P receptor modulator having an ability of regulating growth of airway smooth muscle cells.
5. The pharmaceutical composition for treatment and/or prevention for respiratory diseases according to above-mentioned 3, wherein the S1P receptor modulator is S1P receptor antagonist having an ability for reducing airway resistance.
6. The pharmaceutical composition for treatment and/or prevention for respiratory diseases according to above-mentioned 3, wherein the S1P receptor modulator is S1P receptor agonist having an ability for increasing airway resistance.
7. A pharmaceutical composition for treatment and/or prevention for airway constriction, bronchial asthma, chronic obstructive pulmonary disease (COPD), pulmonary emphysema, tracheostenosis, diffused panbronchialitis, or bronchitis with infection, connective-tissue diseases or trasnplantation, which comprises the S1P receptor antagonist according to above-mentioned 5 as an active ingredient.
8. The pharmaceutical composition for treatment and/or prevention according to above-mentioned 7, wherein the S1P receptor is EDG-1, EDG-3 or EDG-5.
9. The pharmaceutical composition for treatment and/or prevention for respiratory diseases according to above-mentioned 5, wherein the S1P receptor antagonist is an antagonist for EDG-1, EDG-3 or EDG-5.
10. The pharmaceutical composition for treatment and/or prevention for respiratory diseases according to above-mentioned 9, wherein the S1P receptor antagonist is an EDG-5 antagonist.
11. The pharmaceutical composition for treatment and/or prevention for respiratory diseases according to above-mentioned 10, wherein the EDG-5 antagonist is a pyrazolopyridine compound represented by formula (II)

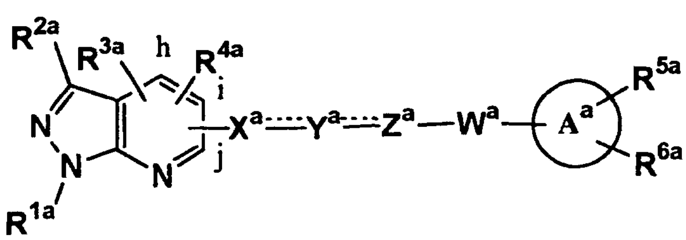

(II)

(wherein all symbols have the same meanings as described hereinafter.) or a nontoxic salt thereof.

12. The pharmaceutical composition for treatment and/or prevention for respiratory diseases according to above-mentioned 4, wherein the S1P receptor modulator having an ability of regulating growth of airway smooth muscle cells is an EDG-5 antagonist.

13. A pharmaceutical composition for treatment and/or prevention for chronic bronchial asthma, lymphangioleiomyomatosis, adult respiratory distress syndrome (ARDS), chronic obstructive pulmonary disease (COPD), interstitial pneumonia, lung cancer, hypersensitivity pneumonitis, diffused panbronchialitis or idiopathic interstitial pneumonia, which comprises the EDG-5 antagonist having an ability of regulating growth of airway smooth muscle cells according to above-mentioned 12 as an active ingredient.
14. The pharmaceutical composition for treatment and/or prevention according to above-mentioned 13, wherein the EDG-5 antagonist is the pyrazolopyridine compound represented by formula (II) according to above-mentioned 11 or a nontoxic salt thereof.
15. A method for measuring airway resistance in mammals characterized by using the S1P receptor agonist having an ability for increasing airway resistance according to above-mentioned 6.
16. A method for measuring airway resistance in mammals according to above-mentioned 15, which comprises a process of S1 P inhalation.
17. A method for screening for compounds having an ability for reducing airway resistance characterized by using the method according to above-mentioned 16. and
18. A compound having an ability for reducing airway resistance obtained by using the method according to above-mentioned 17.

In the present invention, S1P means sphingosine-1-phosphate represented by formula (I).

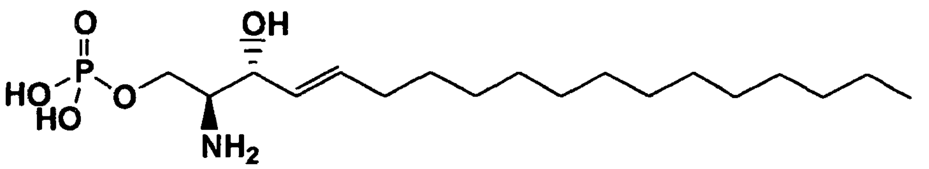

(I)

As the S1P receptor agonists, whatever activates S1P receptor is allowed. As the S1P receptor agonists, the compound obtained naturally or unnaturally may be included. Among the S1P receptor agonists, agonists for EDG-1, 3, 5, 6 and 8 are preferred. Particularly, S1P itself, EDG-3 agonists and EDG-5 agonists are preferred.
S1P receptor agonists are useful in measuring airway resistance in mammals, because they have an ability for inducing airway obstruction by inhalation.
As the S1P receptor antagonists, whatever inactivates S1P receptor is allowed. As the S1P receptor antagonists, the compound obtained naturally or unnaturally may be included. Among the S1P receptor antagonists, antagonists for EDG-1, 3, 5, 6 and 8 are preferred. Particularly, EDG-3 antagonists and EDG-5 antagonists are preferred.
S1P receptor antagonists are useful in treating and/or preventing for diseases that is caused by airway constriction and obstruction, for example, airway constriction, bronchial asthma, chronic obstructive pulmonary disease (COPD), pulmonary emphysema, tracheostenosis, diffused panbronchialitis, and bronchitis with infection, connective-tissue diseases or trasnplantation etc., because they have an ability for reducing airway resistance.
EDG-5 antagonists are useful in treating and/or preventing for diseases that is accompanied by growth of airway smooth muscle cells, for example, chronic bronchial asthma, lymphangioleiomyomatosis, adult respiratory distress syndrome (ARDS), chronic obstructive pulmonary disease (COPD), interstitial pneumonia, lung cancer, hypersensitivity pneumonitis, diffused panbronchialitis, idiopathic interstitial pneumonia etc., because they have an ability for inhibiting growth of airway smooth muscle cells.
In the present invention, as the EDG-5 antagonists, whatever inactivates EDG-5 receptor is allowed. For example, the compound represented by formula (II) or the non-toxic salts thereof are preferred.

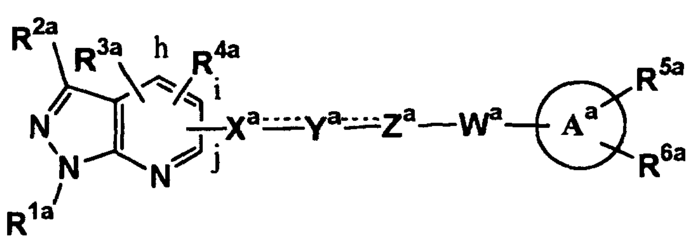

(II)

[wherein $R^{1a}$ represents hydrogen, C1-8 alkyl or $-COR^{7a}$ (wherein $R^{7a}$ represents C1-8 alkyl, optionally substituted aryl, optionally substituted aralkyl, C1-6 alkoxy, optionally substituted aryloxy or optionally substituted aralkyloxy);
$R^{2a}$ represents C1-8 alkyl or optionally substituted aryl;

$R^{3a}$ represents C1-8 alkyl, C1-6 alkoxy, C2-6 alkoxycarbonyl, haloalkyl, C3-7 cycloalkyl or optionally substituted aryl;

$R^{4a}$ represents hydrogen or C1-8 alkyl;

$R^{5a}$ and $R^{6a}$,each independently, represents hydrogen, C1-8 alkyl, C1-6 alkoxy, C2-6 alkoxycarbonyl, carboxyl, C2-6 alkynyl, halogen, cyano, nitro, haloalkyl, C1-8 alkylamino, di(C1-8 alkyl)amino, acyl, hydroxy, optionally substituted aryloxy, optionally substituted aralkyloxy, optionally substituted aryl, optionally substituted aryl, optionally substituted aralkyl, alkoxyalkyl or - $CONHR^{8a}$ (wherein $R^{8a}$ represents optionally substituted aryl or optionally substituted aralkyl);

$X^a$ represents -N($R^{9a}$)- (wherein $R^{9a}$ represents hydrogen, C1-8 alkyl or -$NHR^{10a}$ (wherein $R^{10a}$ represents carboxyl or C2-6 alkoxycarbonyl)), -O-, - N=, -CH= or -CH($R^{11a}$)- (wherein $R^{11a}$ represents hydrogen or C1-8 alkyl);

$Y^a$ represents -N($R^{12a}$)- (wherein $R^{12a}$ represents hydrogen, C1-8 alkyl, optionally substituted aralkyl, C2-6 alkoxycarbonyl, optionally substituted aryloxycarbonyl, optionally substitutedaralkyloxycarbonyl or -$CONHR^{13a}$ (wherein $R^{13a}$ represents optionally substituted aryl or optionally substituted aralkyl)), =N-, -$CH_2$-, =CH-, -O-, -CO- or bond;

$Z^a$ represents -CO-, -CS-, -$CH_2$-, -O- or bond;

$W^a$ represents -N($R^{14a}$)- (wherein $R^{14a}$ represents hydrogen, C1-8 alkyl, optionally substituted aralkyloxycarbonyl, optionally substituted aryloxycarbonyl or heteroaryl-C1-8 alkyl), -O-, -CO-, -CONH (wherein carbon atom bonds with ring$A^a$), or bond;

----- represents double bond or single bond;

Ring$A^a$ represents optionally substituted aryl, heteroaryl or C3-7 cycloalkyl. ]

The present inventors have found that S1P increases airway resistance in guinea pig *in vivo* (Example 1). As prior art which indicates a relation between S1P and airway, there is only a report which discloses *in vitro* experiment using ASM cells previously described (Ammit et al., FASEB J. March 20, 2001). The increase of airway resistance by S1P in the present invention is never indicated from this prior art.

Direct action of S1P on airway resistance was first ascertained by the present invention which discloses an increase of airway resistance is observed when low dose of S1P is inhaled to the airway.

This action was observed significantly when S1P was inhaled (10-100 μg/mL) to anesthetized guinea pig (Example 1), and observed slightly when S1P was injected intravenously (Example 2). In general, an increase of airway resistance is supposed to be involved in airway constriction, airway obstruction and finally respiratory diseases. And it has been known that physiologically active factor which increases airway resistance acts as an aggravation factor of respiratory disease (Saishin Naikagaku Taikei, vol.60, "pulmonary emphysema, obstructive pulmonary disease", and vol.60, "bronchial asthma, allergic pulmonary disease", Nakayama Syoten). This action of S1P was not observed when 10% meylon (vehicle of S1P) or sphingosine (negative control of S1P) was used (Reference example 1). And this action of S1P occurred when treatment by S1P was carried out from outside the body. From those, it has been supposed that this action of S1P is via a S1P receptors. As S1P receptor subtypes, EDG-1, 3, 5, 6 and 8 are suggested. And as site of action of S1P, constriction of bronchial smooth muscle and other action of airway obstruction have been supposed, because pretreatment of salbutamol, a bronchodilator, inhibits airway obstruction partially (Example 3).

From experiments using antagonists for EDG-3 and EDG-5, the inhibitory effect of S1P on airway constriction is supposed to be via EDG-3 and EDG-5 (Example 4, 5 and 6).

And EDG-5 antagonists are supposed to be useful in chronic asthma etc. accompanied with airway remodeling, because cell growth of airway smooth muscle cells is via EDG-5 (Example 7).

Moreover, an increase of airway resistance occurs by administration of S1P, so this experimental system which is measuring airway resistance by inhalation or intravenous injection of S1P is useful in screening system for S1P receptor antagonists because it is (1)high sensitivity for testing physiological activities of S1P, (2)carried out in short term, and (3)versatile for compound assessment.

The present inventors found firstly that S1P has a strong effect on increasing airway resistance. And this effect of S1P has been indicated to be constriction of airway smooth muscle and/or physical airway obstruction. And the correlation between S1P and disease symptom was supposed.

[Toxicity]

The compound used in the present invention has low toxicity so that use of it as a pharmaceutical can be considered as safe enough.

INDUSTRIAL APPLICABILITY

[Application to pharmaceuticals]

The S1P receptor antagonists used in the present invention are useful in preventing and/or treating for diseases that is caused by airway obstruction, for example, airway constriction, bronchial asthma, chronic asthma, chronic obstructive pulmonary disease (COPD), pulmonary emphysema, tracheostenosis, diffused panbronchialitis, and bronchitis with infection, connective-tissue diseases or trasnplantation etc., because they have an ability for binding S1P receptors. Among these S1P receptors, EDG-3 and EDG-5 are involved in these action, so the antagonists for EDG-3 and EDG-5 are useful in preventing and/or treating for these diseases.

And also, the assay system in the present invention using S1P receptor agonists is useful in screening system for S1P receptor antagonists.

The EDG-5 antagonists are useful in preventing and/or treating for diseases that is accompanied by growth of airway smooth cells, for example, chronic bronchial asthma, lymphangioleiomyomatosis, adult respiratory distress syndrome (ARDS), chronic obstructive pulmonary disease (COPD), interstitial pneumonia, lung cancer, hypersensitivity pneumonitis, diffused panbronchialitis, idiopathic interstitial pneumonia etc.

The S1P receptor modulators of the present invention are normally administered systemically or topically, and orally or parenterally for the above purpose.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, or from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

Other drugs may be administered in combination with EDG-5 antagonists for the purpose of complement and/or enhancement of preventing and/or treating effect on chronic asthma.

As other drugs, steroids, beta2 adrenergic receptor stimulants, leukotriene receptor antagonists, thromboxane synthase inhibitors, thromboxane $A_2$ receptor antagonists, mediator release inhibitors, anti-histamine agents, xanthine derivatives, anti-cholinergic agents, cytokine inhibitors, prostaglandins, forskolins, phosphodiesterase inhibitors, elastase inhibitors, metalloproteinase inhibitors, expectorant drugs, antibiotics etc. are given.

As steroids for external use, for example, clobetasol-17-propionate, diflorasone diacetate, fluocinonide, mometasone furoate, betamethasone dipropionate, betamethasone butyrate propionate, betamethasone valerate, difluprednate, budesonide, diflucortrone valerate, amcinonide, halcinonido, dexamethasone, dexamethasone propinate, dexamethasone valerate, dexamethasone acetate, hydrocortisone acetate, hydrocortisone butyrate, hydrocortisone butyrate propionate, deprodone propionate, predonisolone valerate acetate, fluocinolone acetonide, beclometasone dipropionate, triamcinolone acetonide, flumetasone pivalate, alclometasone dipropionate, clobetasone butyrate, prednisolone, beclometasone dipropionate, fludroxycortide etc. are given.

As steroids for internal use or injection, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butyrate acetate, prednisolone sodium phosphate, halopredone acetate, methylprednisolone, methylprednisolone acetate, methylprednisolone sodium, succinate, triamcinolone, triamcinolone acetate, triamcinolone acetonide, dexamethasone, dexamethasone acetate, dexamethasone sodium phosphate, dexamethasone palmitate, paramethasone acetate, betamethasone etc. are given.

As steroids for inhalation, beclometasone dipropionate, fluticasone propionate, budesonide, flunisolide, triamcinolone, ST-126P, ciclesonide, dexamethasone palmitate, mometasone furoate, sodium prasterone sulfate, deflazacort, methylprednisolone suleptanate, methylprednisolone sodium succinate etc. are given.

As beta2 adrenergic receptor stimulants, for example, fenoterol hydrobromide, salbutamol sulfate, terbutaline sulfate, formoterol fumarate, salmeterol xinafoate, isoproterenol sulfate, orciprenaline sulfate, clorprenaline hydrochloride, epinephrine, trimetoquinol hydrochloride, hexoprenalinemesyl sulfate, procaterol hydrochloride, tulobuterol hydrochloride, tulobuterol, pirbuterol hydrochloride, clenbuterol hydrochloride, mabuterol hydrochloride, ritodrine, hydrochloride, bambuterol, dopexamin hydrochloride, meluadrine tartrate, AR-C68397, levosalbutamol, R,R-formoterol, KUR-1246, KUL-7211, AR-C89855, S-1319 etc. are given.

As leukotriene receptor antagonists, for example, pranlukast hydrate, montelukast sodium, zafirlukast, seratrodast, MCC-847, KCA-757, CS-615, YM-158, L-740515, CP-195494, LM-1484, RS-635, A-93178, S-36496, BIIL-284, ONO-4057 etc. are given.

As thromboxane synthase inhibitors, for example, ozagrel hydrochloride, imitrodast sodium etc. are given.

As thromboxane $A_2$ receptor antagonists, for example, seratrodast, ramatroban, domitroban carcium dihydrate, KT-2-962 etc. are given.

As mediator release inhibitors, for example, tranilast, sodium cromoglicate, amlexanox, repirinast, ibudilast, tazanolast, pemirolast potassium etc. are given.

As anti-histamine agents, for example, ketotifen fumarate, mequitazine, azelastine hydrochloride, oxatomide, terfenadine, emedastine difumarate, epinastine hydrochloride, astemizole, ebastine, cetirizine, bepotastine, fexofenadine, lolatadine, desloratadine, olopatadine hydrochloride, TAK-427, ZCR-2060, NIP-530, mometasone furoate, mizolastine, BP-294, andolast, auranofin, aclivastine etc. are given.

As xanthine derivatives, for example, aminophylline, theophylline, doxophylline, cipamfylline, diprophylline etc. are given.

As anti-cholinergic agents, for example, ipratropium bromide, oxitropium bromide, flutropium bromide, cimetropium bromide, temiverine, tiotropium bromide, revatropate (UK-112166) etc. are given.

As cytokine inhibitors, for example, suplatast tosilate (proprietary name : IPD) etc. are given.

As prostaglandin (hereinafter, abbreviated to as PG), PG receptor agonists, PG receptor antagonists etc. are given.

As PG receptors, PGE receptors (EP1, EP2, EP3, EP4), PGD receptors (DP, CRTH2), PGF receptors (FP), PGI receptors (IP), TX receptors (TP) etc. are given.

As phosphodiesterase inhibitors, for example, the PDE4 inhibitors such as rolipram, CILOMILAST (proprietary name : Ariflo), Bay19-8004, NIK-616, Roflumilast (BY-217), cipamfylline (BRL-61063), atizoram (CP-80633), SCH-351591, YM-976, V-11294A, PD-168787, D-4396, IC-485 are given.

As elastase inhibitors, for example, ONO-5046, ONO-6818, MR-889, PBI-1101, EPI-HNE-4, R-665, ZD-0892, ZD-8321, GW-311616, AE-3763 etc. are given.

As expectorant drugs, for example, foeniculated ammonia spirit, sodium hydrogen carbonate, bromhexine hydrochloride, carbocysteine, ambroxol hydrochloride, sustained preparation of ambroxol hydrochloride, methylcysteine hydrochloride, acetylcysteine, L-ethylcysteine hydrochloride, tyloxapol etc. are given.

The compound of the present invention may be administered in the composition of, for example, solid compositions, liquid compositions or other compositions each for oral administration, or injections, liniments or suppositories, each for parenteral administration.

Solid compositions for oral administration include compressed tablets, pills, capsules, powders and granules. Capsules include hard capsules and soft capsules.

In such solid compositions, one or more of the active substance(s) may be used as it stands or as pharmaceuticals by the law of the art in combination with diluting agent (lactose, mannitol, glucose, microcrystallite cellulose, starch etc.), binder (hydroxypropylcellulose, polyvinylpyrrolidone, magnesium aluminometasilicate etc.), disintegrants (cellulose calcium glycolate etc.), lubricants (magnesium stearate etc.), stabilizer and solubilizing agent (glutamic acid, aspartic acid etc.) etc. And it may be coated with a coating agents (sucrose, gelatin, hydroxypropyl cellulose, hydroxypropyl methylcellulose phthalate etc.), or with two or more layers, if necessary. Furthermore, capsules made of a substance which can be absorbed in the body, for example, gelatin, are included.

Liquid compositions for oral administration include pharmaceutically acceptable solutions, suspensions, emulsions, syrups and elixirs. In such liquid compositions, one or more of the active substance(s) may be solved, suspended or emulsified in generally used inert diluent(s) (purified water, ethanol or mixtures thereof etc.). The compositions may comprise, in addition to the inert diluent, humectants, suspending agents, emulsifying agent, sweetening agents, flavoring agents, aromatic agents preservatives and buffer agents.

Injections for parenteral administration include aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol such as ethanol, or a mixture thereof. Moreover, these injections may comprise some additives, such as stabilizing agents, solution adjuvants (such as glutamic acid, aspartic acid or POLYSORBATE80 (registered trade mark), etc.), suspending agents, emulsifying agents, soothing agent, buffering agents, preservative. They may be sterilized at a final step, or may be prepared and compensated according to sterile methods. They may also be manufactured in the form of sterile solid forms, for example, freeze-dried products, which may be dissolved in sterile water or some other sterile diluent(s) for injection immediately before use.

The other compositions for parenteral administration include liquid compositions for external use, ointments, embrocations, inhalations, sprays, suppositories and pessaries for vaginal administration which comprise one or more of the active substance(s) and may be prepared by methods known per se.

Spray may comprise in addition to a generally used diluent, a stabilizer such as sodium bisulfite and an isotonization buffer such as sodium chloride, sodium citrate or citric acid. The preparation process of sprays is described in detail in, for example, U.S. Patent No. 2,868,691 and 3,095,355.

BRIEF DESCRIPTION OF THE DRAWINGS

Figure.1 is a graph which shows an increase of airway resistance of rat induced by S1P inhalation.

Figure.2 is (A) a graph which shows an increase of airway resistance of guinea pig induced by S1P inhalation, and (B) a graph which shows an increase of airway resistance of guinea pig induced by histamine inhalation.
Figure.3 is a graph which shows an effect of sphingosine inhalation on airway of guinea pig.
Figure. 4 is (A) a graph which shows an increase of airway resistance of guinea pig induced by intravenous injection of S1P, and (B) a graph which shows an increase of guinea pig induced by intravenous injection of histamine.
Figure.5 is a graph which shows an effect of administration of salbutamol before S1P administration.
Figure.6 is a graph which shows an inhibitory effect of EDG-5 antagonist on isolated trachea constriction of guinea pig induced by S1P.
Figure.7 is a graph which shows an effect of EDG-5 antagonist against increase of airway resistance of guinea pig induced by S1P.

BEST MODE FOR CARRYING OUT THE INVENTION

Hereinafter, the present invention will be described by reference example and example. However, that the present invention is not limited thereto.

Example 1 : Determination of the airway resistance by using anesthetized guinea pig

The measurement of guinea pig airway resistance was carried out by the modified Konzett and Rössler method (Naunyn-Schmied. Arch. Exp. Path. Pharmak. 195, 71-). Male Hartley guinea pigs (5 to 7 weeks-old, 300 to 500g body weight in use) were anesthetized by pentobarbital (50 mg/kg intraabdominal dosage), and fixed at dorsal position. Then, trachea and jugular were cannulated. Airway was managed by a respirator (5 mL/stroke, 60 strokes/min) and air road was diverged with a T-tube and connected to broncho-spasm-transducer and airway resistance was measured. To guinea pigs, gallamine (1 mg/kg, i.v.) was administered and immobilized. In this connection, acetylcholine was administered (30 μg/kg, i.v.) before inhalation of a compound in order to ascertain that that there was no considerable difference in sensitivity of transient airway constriction between individual animals, and then, animals which have coordinative sensitivity were used.
S1P (Alexis) was vaporized and inhaled with supersonic wave type nebulizer (Omron NE U-07) for five minutes (Jpn. J. Pharm. 50, 445-466 (1989)). Complete obstruction (total area) was calculated as area as follows ; deduct the value of airway resistance at startup of S1P inhalation from the value of airway resistance that is obtained at the time of clamping the tube of tracheal cannula side, and obtained value is multiplied by 10 minutes (5 minutes for inhalation and 5 minutes for post-inhalation). The rate of airway obstruction [(AUC / total area) %] by compound administration was calculated as follows ; the AUC of 10 minutes (5 minutes for inhalation and 5 minutes for post-inhalation) from start of the inhalation divides by complete obstruction (total area). S1P (0.01 to 1 mg/mL) was inhaled to anesthetized guinea pig and airway resistance was measured.
In consequence, strong increase of airway resistance was observed at the time of administration of 0.1 to 1 mg/mL. Figure.1 shows the result of administration of 0.1 mg/mL. Increase of airway resistance were observed persistently and irreversibly when inhalation of S1P was stopped. The increasing effect on airway resistance by S1P inhalation was dose-dependently, and it was at the same level (convert molecular weight of S1P and histamine to number of moles) as inhalation of histamine (Figure 2(A), (B), 0.01 to 1 mg/mL).

Reference example 1 : Determination of the airway resistance by inhalation of sphingosine and 10% Meylon

The reference example was carried out using 10% meylon and sphingosine (Matreya, 0.01 to 1 mg/mL) with method described in example 1. In consequence, increase of airway resistance was not observed in group of 10% meylon and in group of sohingosine (Figure 3).

Example 2 : Determination of the airway resistance induced by intravenous administration of S1P

Male guinea pigs (Standard Hartley, 5 to 7 weeks-old, 300 to 500g body weight in use) were anesthetized by pentobarbital (50 mg/kg intraabdominal dosage), and fixed at dorsal position. Then, trachea and jugular were cannulated. S1P was injected intravenously, and airway resistance was measured as described in Example 1. Increase of airway resistance was observed in S1P administration group (Figure 4(A)). But it was slight compared with that of histamine (Figure 4(B)).

Example 3 : Effect of pre-treatment with salbutamol on airway resistance

The effect of pretreatment of salbutamol (100 μg/mL, i.v.), a bronchodilator on airway resistance was confirmed

as in described in Example 1.

In consequence, the increase of airway resistance induced by S1P was inhibited only partially by the pretreatment of salbutamol (100 μg/mL, i.v.). Therefore it was suggested that the site of action of S1P is a constriction of bronchial smooth muscle cells and airway obstruction other than it (Figure 5).

Example 4 : Evaluation of antagonistic activity for EDG-1, 3 and 5

Chinese hamster ovary (CHO) cells which expressed excessively human EDG-5 gene were cultured using a Ham's F12 medium (GIBCO BRL) containing 10% FBS (fetal bovine serum), penicillin/streptomycin, and blasticidin (5 μg/ml). Cultured cells were incubated in a Fura2 (5 μM) -AM solution [Ham's F12 medium containing FBS (10%), HEPES buffer (20 mM, pH7.4), and probenecid (2.5 mM)] at 37°C for 60 minutes. Then washed once with a Hank's solution (2.5 mM) containing probenecid and immersed into the same solution. A plate was set on a fluorescent drug screening system, and measured for 30 seconds with no stimulation, a solution of a compound (dimethyl sulfoxide solution of 1 nM to 10 μM at the final concentration) to be tested was added. After lapse of 5 minutes, S1P (final concentration : 100 nM) was added, the intracellular calcium ion concentration before and after the addition was measured every 3 seconds (excitation wave length: 340 nm and 380 nm; fluorescent wave length: 500 nm). The antagonizing activity of EDG-5 was calculated as an inhibition rate (%) by the following equation, wherein the peak value of S1P (final concentration: 100 nM) in a well into which DMSO containing no Compound was added was regarded as a control value (A), and in the cells treated with the compound the difference (B) between the value before addition of the compound and that after the addition was obtained and compared with the control value.

$$\text{Inhibition rate (\%)} = ((A-B) / A) \times 100$$

The $IC_{50}$ value was calculated as a concentration of the compound of the present invention which showed 50% inhibition. In addition, the evaluation of antagonist for EDG-1 and EDG-3 was carried out using cells which expresses excessively human EDG-1 or EDG-5 and using method as in EDG-5. Table 1 shows activity of compound 1: (N-(1H-1,3-dimethyl-4-isopropylpyrazolo[3,4-b]pyridine-6-yl)amino-N'-(3,5-dichloropyridine-4-yl)urea) which is EDG-5 antagonist as example.

Table 1

| | IC50 (μM) | | |
|---|---|---|---|
| | EDG-1 | EDG-3 | EDG-5 |
| Compound 1 | more than 10 | more than 10 | 0.01 |

Example 5 : Inhibitory effect of an antagonist for EDG-5 and EDG-3 against constriction activity of S1P of isolated trachea of guinea pig

The trachea of euthanized male Hartley ginea pig was removed and immersed quickly in Krebs-Henseleit solution (112 mmol/L sodium chloride, 5.9 mmol/L potassium chloride, 2.0 mmol/L calcium chloride, 1.2 mmol/L magnesium chloride, 1.2 mmol/L monobasic sodium phosphate, 25.0 mmol/L sodium hydrogen carbonate $NaHCO_3$, 11.5 mmol/L glucose, 4°C). A removed tissue was hooked as helical sample in magnus tube (volume : 10 mL) filled with Krebs-Henseleit solution (37±1°C, [aerated with 95% $O_2$ + 5% $CO_2$]). After stabilizing for 60 minutes in tensioned condition with 1 g, contraction was measured by recorder from force displacement transducer via distortion amplifier. The contraction of control was obtained by high concentration solution of potassium chloride (sodium chloride in Krebs-Henseleit solution was all exchanged to potassium chloride), or by stimulation with leukotriene D4 (LTD4) or histamine. After contraction by S1P, EDG-5 antagonist was added. Washing process of a specimen was repeated just in time, after hooked in magnus tube.

In result of observation of action of S1P (10 μmol/L) on guinea pig trachea, S1P induced very slow contraction, and rate of this contraction was 3 to 5 fold slower than other constricting drug (LTD4, histamine etc.), and it was taken as long as about 30 to 60 minutes to plateau (Figure 6).

Next, the actions of various S1P agonist was tested. Compound 1 (N-(1H-1,3-dimethyl-4-isopropylpyrazolo [3,4-b]pyridine-6-yl)amino-N'-(3,5-dichloropyridine-4-yl)urea) ; an specific antagonist for EDG-5 inhibited constriction induced by S1P (Figure 6). From these, it was supposed that the receptor concerning in the airway constriction of S1P was EDG-5.

In proviso, intervention of action of EDG-3 is accepted besides EDG-5 for similar examination with a rat.

Example 6 : Inhibitory effect of an EDG-5 antagonist against increase of airway resistance of anesthetized guinea pig induced by S1P *in vivo*

The measurement of guinea pig airway resistance was carried out by the modified Konzett and Rössler method (Naunyn-Schmied. Arch. Exp. Path. Pharmak. 195, 71- ). Male Hartley guinea pigs (5 to 7 weeks-old, 300 to 500g body weight in use) were anesthetized by pentobarbital (50 mg/kg intraabdominal dosage), and fixed at dorsal position. Then, trachea and jugular were cannulated. Airway was managed by a respirator (5 mL/stroke, 60 strokes/min) and air road was diverged with a T-tube and connected to broncho-spasm-transducer and airway resistance was measured. To guinea pigs, gallamine (1 mg/kg, i.v.) was administered and immobilized. S1P was vaporized and inhaled with supersonic wave type nebulizer for five minutes (Jpn. J. Pharm. 50, 445-466 (1989)). S1P (0.1 mg/mL) was inhaled to the anesthetized guinea pig, and airway resistance was measured. After a increase of strong airway resistance was recognized, compound 1 was intravenously injected. In consequence, an increase of airway resistance induced by S1P was inhibited, so it was supposed that the increase of airway resistance induced by S1P in vivo is via an EDG-5.

Example 7 : Inhibitory effect of an EDG-5 antagonist against acceleration of DNA synthesis of human airway smooth muscle cells induced by S1P

Normal human airway smooth muscle cells (BMSC; BioWhittaker) were cultured using MEM-$\alpha$ medium (GIBCO) containing 10% FBS (fetal calf serum), gentamicin (50 $\mu$g/mL), amphotericin B (50 ng/mL), and human bFGF, human insulin for 24 hours and deoxybromouridine (BrdU) was added.

Normal human airway smooth muscle cells (BMSC ; BioWhittaker) were plated in 96 well plate using MEM-$\alpha$ medium containing human insulin at density of $1x10^4$ cells/well. After 24 hour incubation, wash 2 times with Hanks solution, and serum-free medium (MEM-$\alpha$/gentamicin (50 $\mu$g/mL), amphotericin B (50 ng/mL)) was added and incubated for 24 hours. S1P was added at the final concentration of 0.03 $\mu$M to 10 $\mu$M, deoxybromouridine (BrdU) was added at the same time, and incubated for 6 hours, and then the activity of BrdU incorporation was measured. As control, equal volume of physiological salt solution, which is vehicle of S1P, was added.

Cells were treated with BrdU, and culture supernatant was discarded. 150 $\mu$L of Cell fixation·DNA denaturation buffer was added to each well and incubated for 30 minutes. Cell fixation·DNA denaturation buffer was removed and blocking buffer (150 $\mu$L) was added. After incubation for 30 minutes, blocking buffer was removed, and peroxidase-labelled anti-BrdU antibody solution (100 $\mu$L) was added and incubated for 30 minutes. After removal of antibody solution, wash process using wash buffer (PBS(-)) (150 $\mu$L) was carried out 3 times. After removal of moisture, reaction substrate (3,3',5,5'-tetramethylbenzidine in 15% DMSO ; 100 $\mu$L) was added. After 15 minutes, 25 $\mu$L of 2N sulfuric acid was added and absorbance at 450 nm was measured.

BSMC was cultured as in described before, compound 1 (final concentration : 0.01 $\mu$M to 10 $\mu$M ) was added just before addition of S1P. After addition of S1P (3 $\mu$M), BrdU was added and cultured for 6 hours more. The activity of BrdU incorporation was measured, in result, EDG-5 antagonists antagonized against S1P.

In cell toxicity assessment using released LDH (lactate dehydrogenase) as an indicator, compound 1 did not enhancement LDH activity in medium.

From these, it was suggested that S1P stimulation induces DNA synthesis of human airway smooth muscle cells and EDG-5 is the associated receptor of it.

## Claims

1. A pharmaceutical composition for treatment and/or prevention for respiratory diseases comprising a sphingosine-1-phosphate (S1P) receptor modulator.

2. A pharmaceutical composition for treatment and/or prevention for airway constriction, bronchial asthma, chronic obstructive pulmonary disease (COPD), pulmonary emphysema, tracheostenosis, diffused panbronchialitis, or bronchitis with infection, connective-tissue diseases or trasnplantation, lymphangioleiomyomatosis, adult respiratory distress syndrome (ARDS), interstitial pneumonia, lung cancer, hypersensitivity pneumonitis or idiopathic interstitial pneumonia, which comprises the sphingosine-1-phosphate (S1P) receptor modulator according to claim 1 as an active ingredient.

3. The pharmaceutical composition for treatment and/or prevention for respiratory diseases according to claim 1, which comprises a S1P receptor modulator having an ability of regulating airway resistance.

4. The pharmaceutical composition for treatment and/or prevention for respiratory diseases according to claim 1,

which comprises a S1P receptor modulator having an ability of regulating growth of airway smooth muscle cells.

**5.** The pharmaceutical composition for treatment and/or prevention for respiratory diseases according to claim 3, wherein the S1P receptor modulator is S1P receptor antagonist having an ability for reducing airway resistance.

**6.** The pharmaceutical composition for treatment and/or prevention for respiratory diseases according to claim 3, wherein the S1P receptor modulator is S1P receptor agonist having an ability for increasing airway resistance.

**7.** A pharmaceutical composition for treatment and/or prevention for airway constriction, bronchial asthma, chronic obstructive pulmonary disease (COPD), pulmonary emphysema, tracheostenosis, diffused panbronchialitis, or bronchitis with infection, connective-tissue diseases or trasnplantation, which comprises the S1P receptor antagonist according to claim 5 as an active ingredient.

**8.** The pharmaceutical composition for treatment and/or prevention according to claim 7, wherein the S1P receptor is EDG-1, EDG-3 or EDG-5.

**9.** The pharmaceutical composition for treatment and/or prevention for respiratory diseases according to claim 5, wherein the S1P receptor antagonist is an antagonist for EDG-1, EDG-3 or EDG-5.

**10.** The pharmaceutical composition for treatment and/or prevention for respiratory diseases according to claim 9, wherein the S1P receptor antagonist is an EDG-5 antagonist.

**11.** The pharmaceutical composition for treatment and/or prevention for respiratory diseases according to claim 10, wherein the EDG-5 antagonist is a pyrazolopyridine compound represented by formula (II)

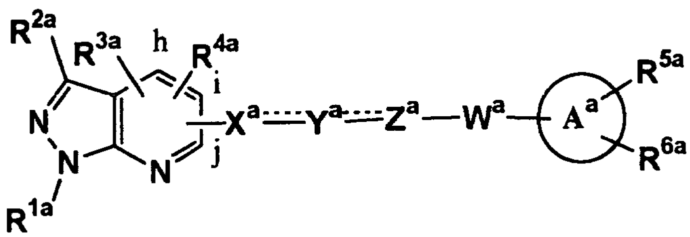

(II)

wherein $R^{1a}$ represents hydrogen, C1-8 alkyl or -$COR^{7a}$, wherein $R^{7a}$ represents C1-8 alkyl, optionally substituted aryl, optionally substituted aralkyl, C1-6 alkoxy, optionally substituted aryloxy or optionally substituted aralkyloxy;

$R^{2a}$ represents C1-8 alkyl or optionally substituted aryl;

$R^{3a}$ represents C1-8 alkyl, C1-6 alkoxy, C2-6 alkoxycarbonyl, haloalkyl, C3-7 cycloalkyl or optionally substituted aryl;

$R^{4a}$ represents hydrogen or C1-8 alkyl;

$R^{5a}$ and $R^{6a}$, each independently, represents hydrogen, C1-8 alkyl, C1-6 alkoxy, C2-6 alkoxycarbonyl, carboxyl, C2-6 alkynyl, halogen, cyano, nitro, haloalkyl, C1-8 alkylamino, di(C1-8 alkyl)amino, acyl, hydroxy, optionally substituted aryloxy, optionally substituted aralkyloxy, optionally substituted aryl, optionally substituted aryl, optionally substituted aralkyl, alkoxyalkyl or - $CONHR^{8a}$, wherein $R^{8a}$ represents optionally substituted aryl or optionally substituted aralkyl;

$X^a$ represents -N($R^{9a}$)-, wherein $R^{9a}$ represents hydrogen, C1-8 alkyl or -$NHR^{10a}$, wherein $R^{10a}$ represents carboxyl or C2-6 alkoxycarbonyl; -O-; -N=; -CH=; or -CH($R^{11a}$)-, wherein $R^{11a}$ represents hydrogen or C1-8 alkyl;

$Y^a$ represents -N($R^{12a}$)-, wherein $R^{12a}$ represents hydrogen, C1-8 alkyl, optionally substituted aralkyl, C2-6 alkoxycarbonyl, optionally substituted aryloxycarbonyl, optionally substitutedaralkyloxycarbonyl or -$CONHR^{13a}$, wherein $R^{13a}$ represents optionally substituted aryl or optionally substituted aralkyl; =N-; -$CH_2$-; =CH-; -O-; -CO-; or bond;

$Z^a$ represents -CO-, -CS-, -$CH_2$-, -O- or bond;

$W^a$ represents -N($R^{14a}$)-, wherein $R^{14a}$ represents hydrogen, C1-8 alkyl, optionally substituted aralkyloxycarbonyl, optionally substituted aryloxycarbonyl or heteroaryl-C1-8 alkyl; -O-, -CO-, -CONH, wherein carbon atom bonds with ring$A^a$; or bond;

----- represents double bond or single bond;
RingA$^a$ represents optionally substituted aryl, heteroaryl or C3-7 cycloalkyl, or
a nontoxic salt thereof.

**12.** The pharmaceutical composition for treatment and/or prevention for respiratory diseases according to claim 4, wherein the S1P receptor modulator having an ability of regulating growth of airway smooth muscle cells is an EDG-5 antagonist.

**13.** A pharmaceutical composition for treatment and/or prevention for chronic bronchial asthma, lymphangioleiomyomatosis, adult respiratory distress syndrome (ARDS), chronic obstructive pulmonary disease (COPD), interstitial pneumonia, lung cancer, hypersensitivity pneumonitis, diffused panbronchialitis or idiopathic interstitial pneumonia, which comprises the EDG-5 antagonist having an ability of regulating growth of airway smooth muscle cells according to claim 12 as an active ingredient.

**14.** The pharmaceutical composition for treatment and/or prevention according to claim 13, wherein the EDG-5 antagonist is the pyrazolopyridine compound represented by formula (II) according to claim 11 or a nontoxic salt thereof.

**15.** A method for measuring airway resistance in mammals **characterized by** using the S1P receptor agonist having an ability for increasing airway resistance according to claim 6.

**16.** A method for measuring airway resistance in mammals according to claim 15, which comprises a process of S1P inhalation.

**17.** A method for screening for compounds having an ability for reducing airway resistance **characterized by** using the method according to claim 16.

**18.** A compound having an ability for reducing airway resistance obtained by using the method according to claim 17.

Figure. 1

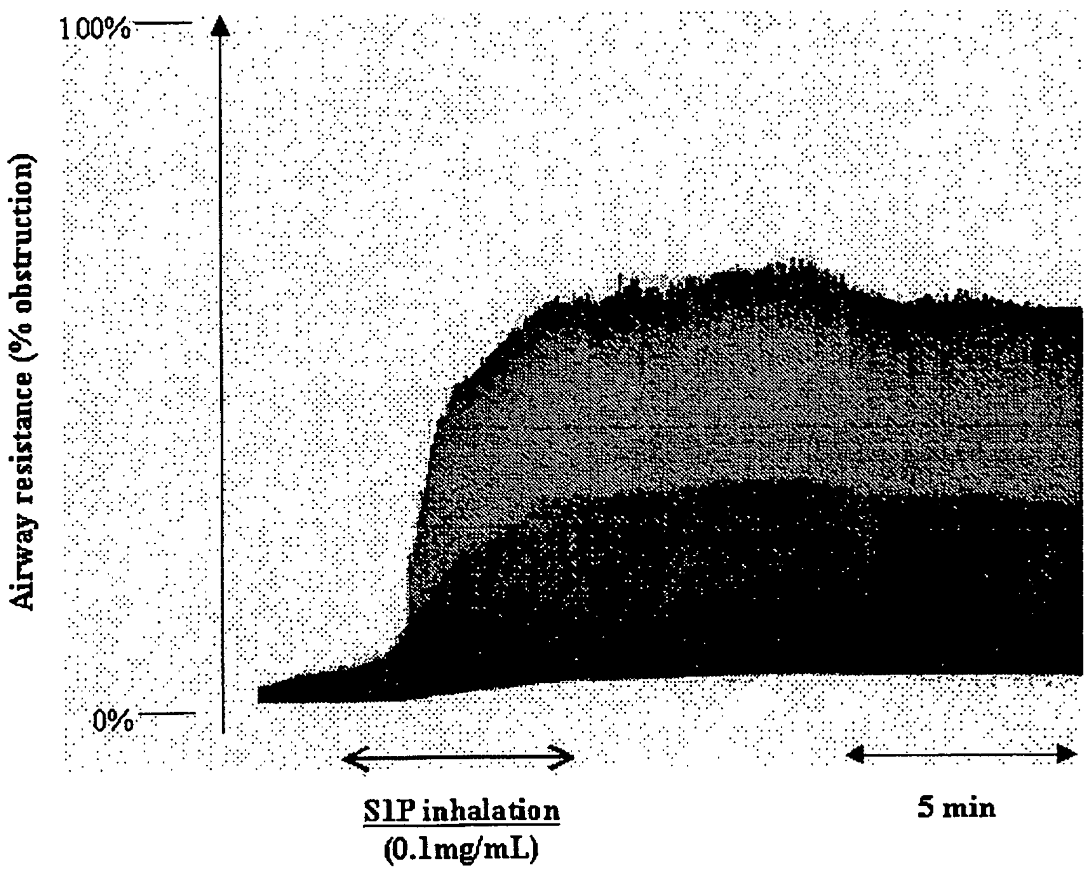
100%
0%
Airway resistance (% obstruction)
S1P inhalation
(0.1mg/mL)
5 min

Figure. 2

**(A) S1P inhalation**

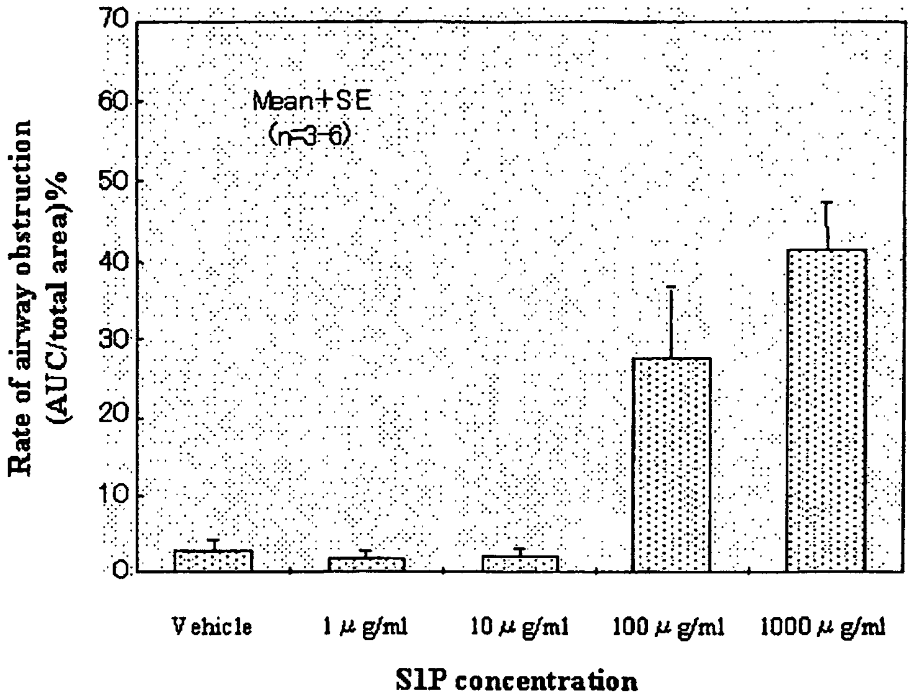


**(B) Histamine inhalation**

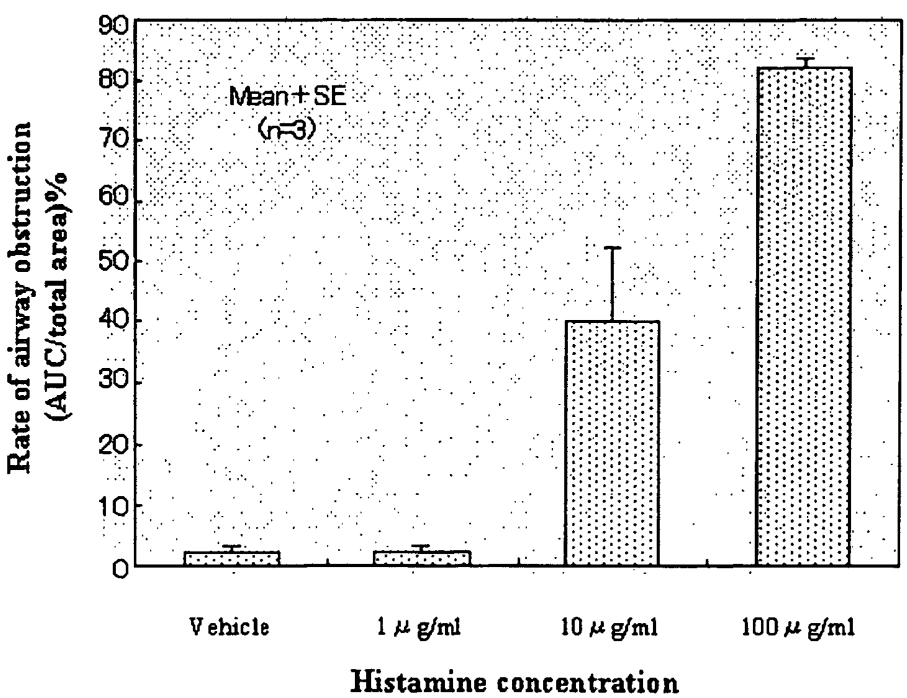

Figure. 3

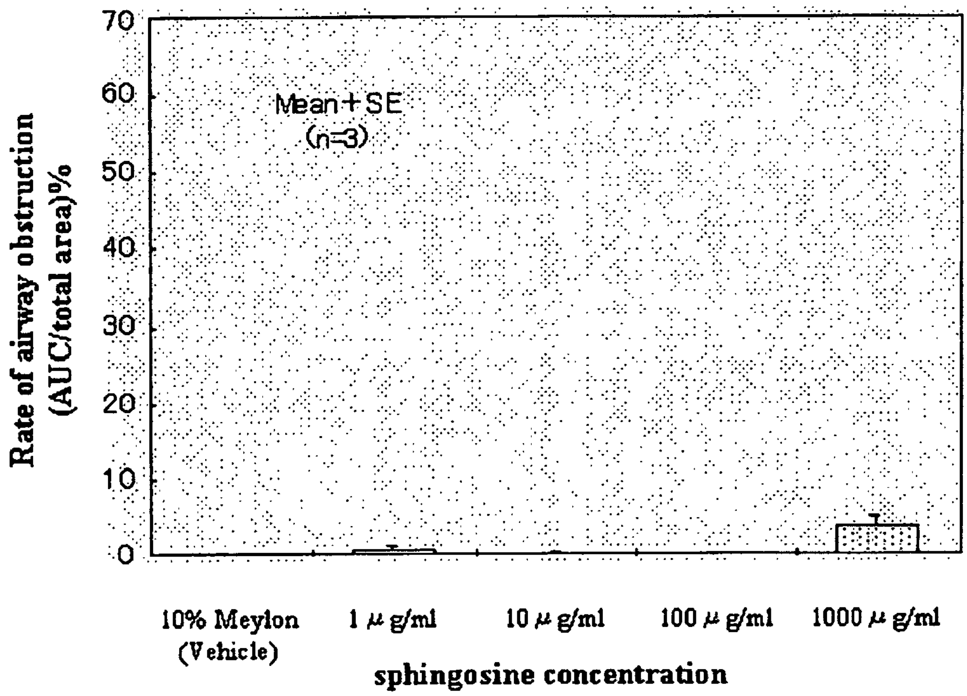
70
60
50
40
30
20
10
0
Mean+SE
(n=3)
Rate of airway obstruction
(AUC/total area)%
10% Meylon
(Vehicle)
1 μ g/ml
10 μ g/ml
100 μ g/ml
1000 μ g/ml
sphingosine concentration

Figure. 4

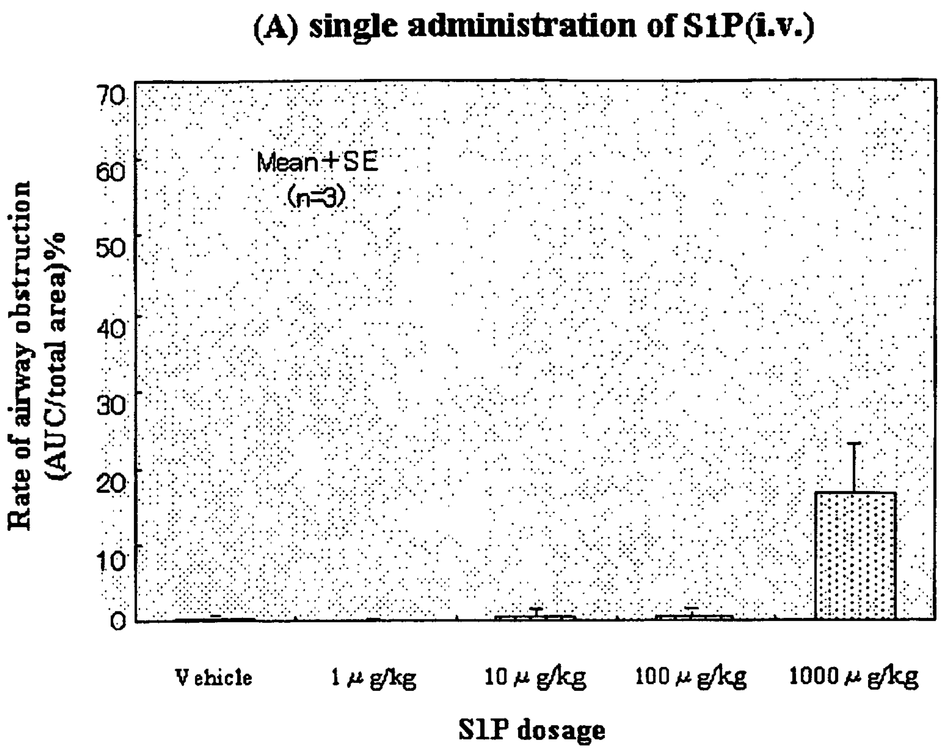
(A) single administration of S1P(i.v.)
Mean+SE
(n=3)
Rate of airway obstruction
(AUC/total area)%
70
60
50
40
30
20
10
0
Vehicle
1 μ g/kg
10 μ g/kg
100 μ g/kg
1000 μ g/kg
S1P dosage

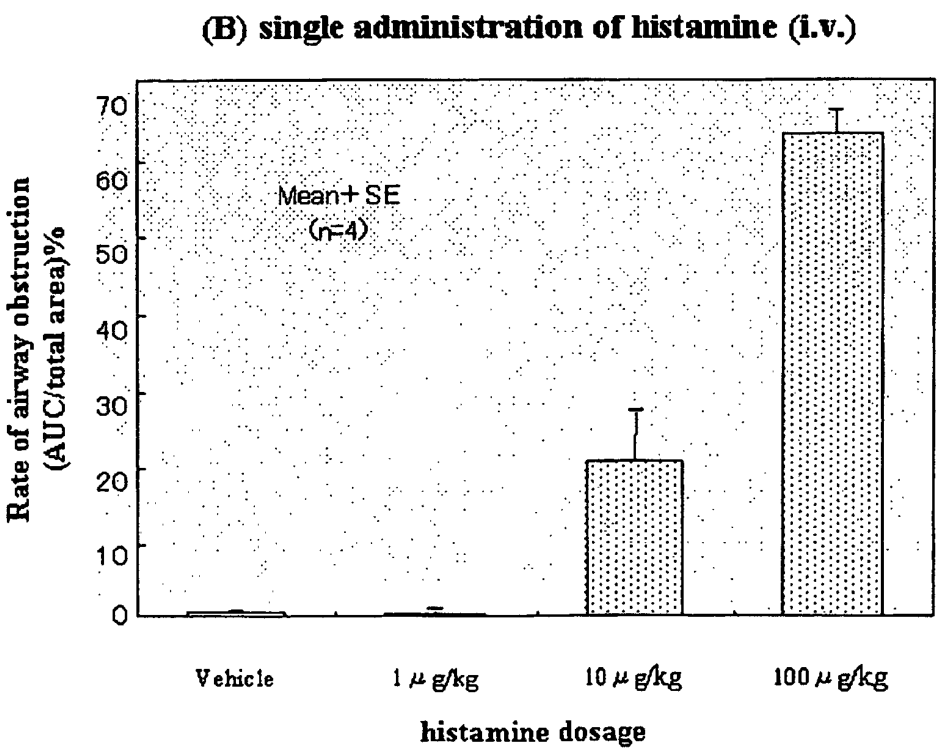
(B) single administration of histamine (i.v.)
Mean+SE
(n=4)
Rate of airway obstruction
(AUC/total area)%
70
60
50
40
30
20
10
0
Vehicle
1 μ g/kg
10 μ g/kg
100 μ g/kg
histamine dosage

Figure. 5

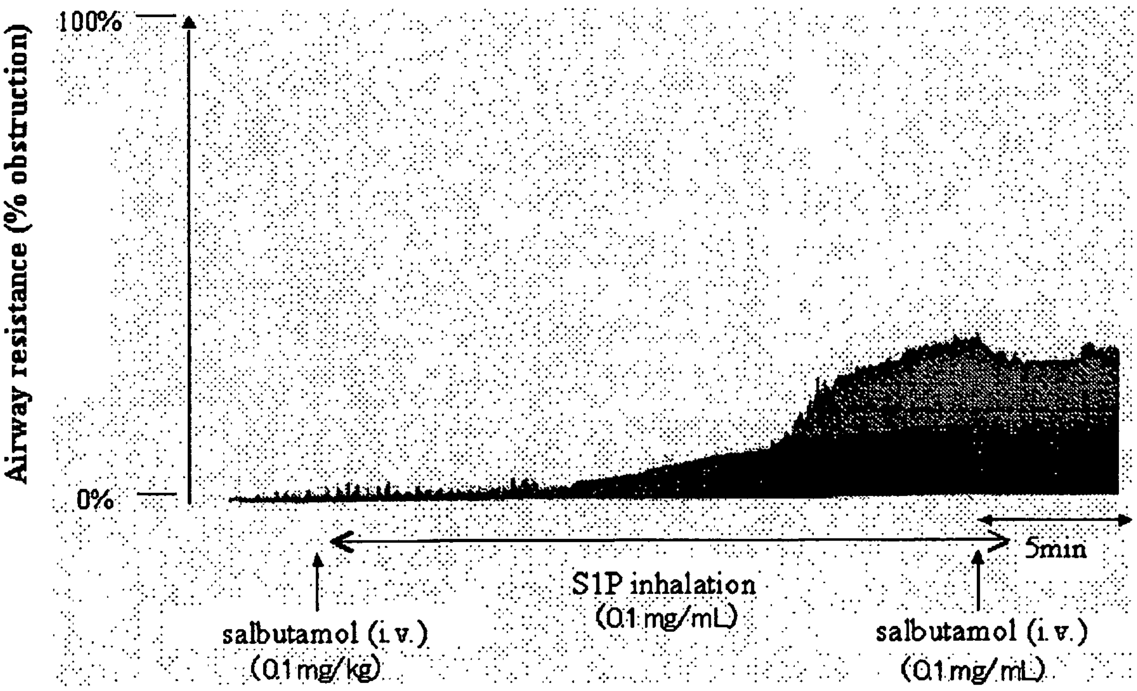
100%
0%
Airway resistance (% obstruction)
5min
S1P inhalation
(0.1 mg/mL)
salbutamol (i.v.)
(0.1 mg/kg)
salbutamol (i.v.)
(0.1 mg/mL)

Figure. 6

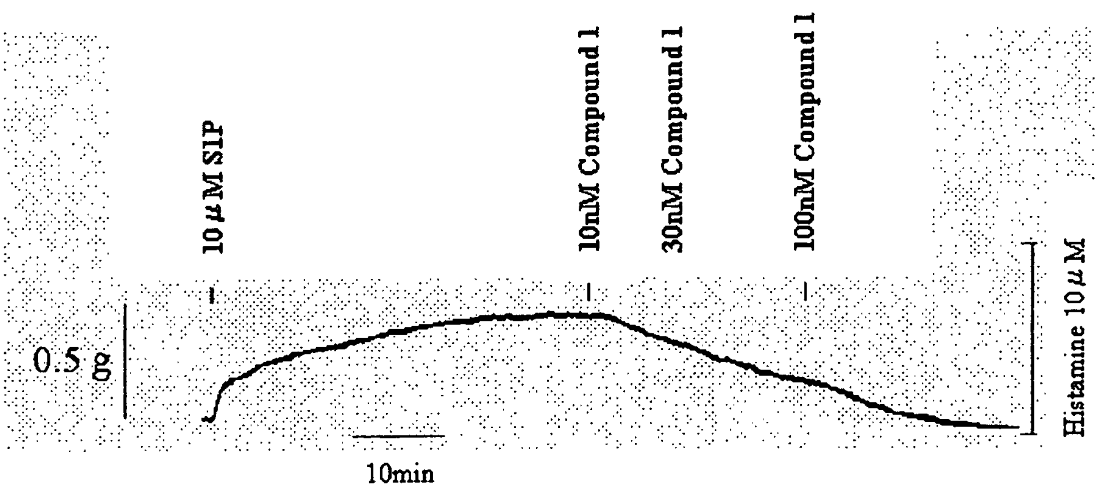
10 μM SIP
10nM Compound 1
30nM Compound 1
100nM Compound 1
0.5 g
10min
Histamine 10 μM

Figure. 7

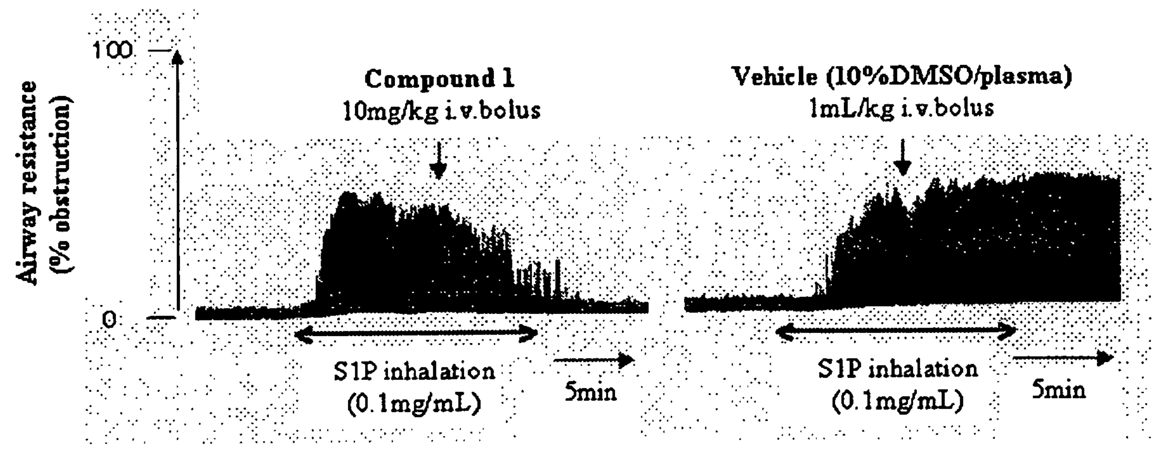
100
0
Airway resistance
(% obstruction)
Compound 1
10mg/kg i.v.bolus
Vehicle (10%DMSO/plasma)
1mL/kg i.v.bolus
S1P inhalation
(0.1mg/mL)
5min
S1P inhalation
(0.1mg/mL)
5min

## INTERNATIONAL SEARCH REPORT

International application No.
PCT/JP02/08926

A. CLASSIFICATION OF SUBJECT MATTER
Int.Cl$^7$ A61K45/00, 31/437, A61P11/00, 11/06

According to International Patent Classification (IPC) or to both national classification and IPC

B. FIELDS SEARCHED

Minimum documentation searched (classification system followed by classification symbols)
Int.Cl$^7$ A61K45/00, 31/437, A61P11/00, 11/06

Documentation searched other than minimum documentation to the extent that such documents are included in the fields searched

Electronic data base consulted during the international search (name of data base and, where practicable, search terms used)
CA(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| P,X | WO 01/98301 A1 (Japan Tobacco Inc.), 27 December, 2001 (27.12.01), Full text & AU 200164313 A | 1-5,9-14 |
| P,X | JP 2002-212070 A (Ono Pharmaceutical Co., Ltd.), 31 July, 2002 (31.07.02), Full text (Family: none) | 1,2,3,4,5 |
| X<br>Y | WO 01/03739 A1 (Ono Pharmaceutical Co., Ltd.), 18 January, 2001 (18.01.01), Full text & EP 1195165 A1 & KR 2002027463 A | 1-4,6,17,18<br>5,7-14 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 December, 2002 (06.12.02) | 24 December, 2002 (24.12.02) |
| Name and mailing address of the ISA/ Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/08926

C (Continuation). DOCUMENTS CONSIDERED TO BE RELEVANT

| Category* | Citation of document, with indication, where appropriate, of the relevant passages | Relevant to claim No. |
|---|---|---|
| X | ALAINA J. AMMIT et al., Sphigosine 1-phosphate modulates human airway smooth muscle cell functions that promote inflammation and airway remodelin in asthma, The FASEB Journal, March 2001, Vol.15, No.7, pages 1212 to 1214 | 1-5,7-10, 12-13 |

Form PCT/ISA/210 (continuation of second sheet) (July 1998)

**INTERNATIONAL SEARCH REPORT**

International application No.
PCT/JP02/08926

**Box I Observations where certain claims were found unsearchable (Continuation of item 1 of first sheet)**

This international search report has not been established in respect of certain claims under Article 17(2)(a) for the following reasons:

1. [x] Claims Nos.: 15-16
because they relate to subject matter not required to be searched by this Authority, namely:
Claims 15 and 16 pertain to diagnostic methods to be practiced on the human body and thus relate to a subject matter which this International Searching Authority is not required, under the provisions of Article 17(2)(a)(i) of the PCT and Rule 39.1(iv) of the Regulations under the PCT, to search

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

INTERNATIONAL SEARCH REPORT

International application No.
PCT/JP02/08926

<Subject of search>

Claims 1 to 10, 12, 13, 17 and 18 relate to remedies for respiratory diseases containing as the active ingredient a compound defined by a desired property of being "a sphingosine-1-phosphate (S1P) receptor controller". Although any compounds having this property are involved in the scopes of claims 1 to 10, 12, 13, 17 and 18, it is considered that only part of the claimed compounds are supported by the description in the meaning as specified in PCT Article 6 and disclosed therein in the meaning as specified in PCT Article 5.

Although the common technical knowledge at the point of the application is taken into consideration, the scope of the compounds having the property of being "a sphingosine-1-phosphate (S1P) receptor controller" cannot be specified. Thus, claim 1 fails to fulfill the requirement of clearness under PCT Article 6 too.

Such being the case, the search was made on the relation between sphingosine-1-photphate receptor controllers and respiratory diseases and remedies for respiratory diseases containing as the active ingredient the compounds specified in claims 11 and 14. Claims 11 and 14 were completely searched.

Form PCT/ISA/210 (extra sheet) (July 1998)